# EUROPEAN PATENT SPECIFICATION

(11) **EP 2 671 546 B1**
(45) Date of publication and mention of the grant of the patent: **14.10.2015**
(21) Application number: 13170520.4
(22) Date of filing: 04.06.2013
(51) Int. Cl.: A61F 5/50

(54) **Apparatus with retainer for treating thumb or finger sucking**
Vorrichtung mit Halter zur Behandlung von Daumen- oder Fingerlutschen
Appareil muni d'un dispositif de retenue pour le traitement de succion du pouce ou du doigt

(30) Priority: 07.06.2012 US 201213491231
(43) Date of publication of application: 11.12.2013
(73) Proprietor: Med et al, Inc, Matthews, NC 28105 (US)
(72) Inventor: Zilber, David, Matthews, NC North Carolina 28105 (US)
(74) Representative: Hargreaves, Timothy Edward

(56) References cited:
- WO-A1-03/009790
- US-A- 5 515 870

## Description

### FIELD OF THE INVENTION

The present invention generally relates to treating chronic thumb sucking, and more particularly, to an apparatus and associated method for discouraging thumb or finger sucking.

### BACKGROUND OF THE INVENTION

Thumb (or finger) sucking at an early age is considered to be a completely normal and natural activity among children, which stems from a very strong sucking reflex that is vital for children's survival. While infants are sucking, which is done as a means to receive nutrition, they experience pleasurable oral sensations, some of which become associated with the fulfillment from food, closeness and tenderness. In most cases, as the child matures, they may find alternative means of receiving the same forms of positive physical and emotional fulfillment.

However, some children look for a way to continue the once necessary and pleasantly soothing experience of nutritional sucking (either breastfeeding or sucking from the bottle), thus forming the thumb (or finger) sucking habit. This habit, if not eliminated by a certain age, may become detrimental to a child's physical, emotional and social development. Several scientific studies and guidelines indicate that children should quit the habit between the ages of three and four, as opposed to the "rule of thumb" approach used by some pediatric dentists; stating that the habit should be eliminated before the arrival of permanent teeth (ages five to six).

There are numerous approaches to treat chronic (habitual) thumb sucking. One such approach is disclosed in U.S. Patent No. 5,515,870, entitled: *Thumb and Finger Sucking Prevention Device.* The '870 patent is directed to an apparatus for the prevention of thumb and finger sucking. The apparatus utilizes a vacuum-breaking device such as a cylinder that is placed over one or more fingers or the thumb and prevents the formation of a sucking vacuum by utilizing air passages in or between the vacuum-breaking cylinder and the thumb or fingers. The vacuum-breaking cylinder is secured to the hand by means of a wristband and a suitable means, such as one or more straps, for connecting the vacuum-breaking cylinder to the wristband. WO 03/009790 is considered to represent the closest prior art, it describes a device for preventing a child's habit of sucking a thumb and/or finger, which comprises first, second and third securing parts and a bonding band communicating the first, second and third securing parts and securing the device to the user's hand.

### SUMMARY OF THE INVENTION

In light of the foregoing background, exemplary embodiments of the present invention provide an improved apparatus and method for discouraging or prevent thumb or finger sucking to break the habit without the use of a substitute pacifier, distasteful chemical compositions, or the restraint of a child's hand or fingers. The apparatus may be worn by a child or other user without discomfort or restraint of normal activity. According to one example aspect of the present invention, an apparatus is provided for discouraging a user from sucking on a digit (finger or thumb) of the user's hand. The apparatus of this example aspect includes a hollow vacuum-breaking device, a pair of straps extending from the vacuum-breaking device, and a retainer.

The vacuum-breaking device includes a base and defining one or more air passages, the vacuum-breaking device being sized to permit insertion of the user's digit into the vacuum-breaking device so that the base of the vacuum-breaking device is nearer to the base of the digit than the tip thereof. The air passages of the vacuum-breaking device prevent the user from establishing a sucking vacuum when the respective digit including the vacuum-breaking device is inserted into the user's mouth.

The straps of the pair of straps define respective pluralities of holes along their length, a pair of the holes including a hole of each of the pluralities being selectable according to a size of the user's hand, the straps being configured to cross when the holes of the pair of holes overlap. The retainer is configured to extend through overlapping holes of the straps to thereby secure the straps together. The retainer also defines an aperture for receiving a wristband securable to the user's wrist to secure the straps to the user's wrist, with the vacuum-breaking device thereby being securable to the user's hand.

In one example, the retainer is rigid, and the holes of the straps are slightly deformable to allow the retainer to be threaded through the overlapping holes and secured in place.

In one example, the retainer includes a bottom section and an opposing top section each of which is larger than the holes of the straps, and an intermediate section between the bottom and top sections that is approximately the same size as or smaller than the holes of the straps. In a further example, the top section may be inwardly squeezable from a first orientation to a second orientation to allow the top section to be threaded through the overlapping holes. In this further example, the top section may be capable of thereafter returning to the first orientation to secure the retainer in place.

In one example, the apparatus may further include a hollow choke including a base that is fixed in size and smaller than that of the vacuum-breaking device. In this example, the vacuum-breaking device and choke may be sized to permit insertion of the choke into a bottom section of the vacuum-breaking device proximate the base thereof, with the vacuum-breaking device and choke thereby being assembled. The vacuum-breaking device and choke may also be sized to permit insertion of the user's digit into the assembled vacuum-breaking device and choke so that the bases of the vacuum-breaking device and choke are nearer to the base of the digit than the tip thereof.

In one example, the vacuum-breaking device may have a conical-frustum shape including the base and an opposing top which has a diameter smaller than that of the base of the vacuum-breaking device. In this example, the choke may have a conical-frustum shape including the base and an opposing top which has a diameter larger than that of the base of the choke, with the diameter of the base of the choke being smaller than that of the base of the vacuum-breaking device.

In one example, the choke may include a plurality of protrusions corresponding to a plurality of air passages of the vacuum-breaking device. In this example, the protrusions may be configured to extend out from respective air passages of the assembled vacuum-breaking device and choke to thereby secure the choke in place.

According to other aspects of exemplary embodiments of the present invention, other apparatuses are provided for discouraging a user from sucking on a digit (finger or thumb) of the user's hand. Exemplary embodiments of the present invention therefore provide improved apparatuses for discouraging a user from sucking on a digit (finger or thumb) of the user's hand. As indicated above and explained below, exemplary embodiments of the present invention may provide one or more advantages over existing techniques.

### BRIEF DESCRIPTION OF THE DRAWINGS

Having thus described the invention in general terms, reference will now be made to the accompanying drawings, which are not necessarily drawn to scale, and wherein:
FIG. 1 illustrates various views of an apparatus according to example embodiments of the present invention fitted to a user's hand to discourage thumb sucking, including a front/back view (FIG. 1a) and side view (FIG. 1b);
FIG. 2 illustrates various views of a vacuum-breaking device of the apparatus of one example embodiment, including perspective views (top-down, FIG. 2a; bottom-up, FIG. 2b), top view (FIG. 2c) and front view (FIG. 2d);
FIG. 3 illustrates various views of a choke of the apparatus of one example embodiment, including perspective views (top-down, FIG. 3a; bottom-up, FIG. 3b), top view (FIG. 3c) and front view (FIG. 3d);
FIG. 4 illustrates various views of an assembled vacuum-breaking device and choke of the apparatus of one example embodiment, including a perspective view (FIG. 4a), top view (FIG. 4b), front view (FIG. 4c), and cross-section view (FIG. 4d);
FIG. 5 illustrates various views of a retainer of the apparatus of one example embodiment, including a perspective view (FIG. 5a), top view (FIG. 5b) and front view (FIG. 5c);
FIG. 6a illustrates various views of a portion of an example vacuum-breaking device assembled with a retainer, including a top view (FIG. 6a), top-down perspective views (perspective view, FIG. 6b; exploded perspective view, FIG. 6c), bottom-up perspective view (FIG. 6d) and side view (FIG. 6e);
FIG. 7 illustrates various views of a retainer of the apparatus of one another example embodiment, including a perspective view (FIG. 7a), top view (FIG. 7b) and front view (FIG. 7c); and
FIG. 8 illustrates various views of a multi-component retainer of the apparatus of another example embodiment, including perspective views of the retainer (FIG. 8) and its components (FIGS. 8b, 8c and 8d), and front views of the retainer (FIG. 8e) and its components (FIGS. 8f, 8g and 8h).

### DETAILED DESCRIPTION OF THE INVENTION

The present invention now will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. For example, references may be made herein to directions and orientations including vertical, horizontal, diagonal, right and left, front and back; it should be understood, however, that any direction and orientation references are simply examples and that any particular direction or orientation may depend on the particular object, and/or the orientation of the particular object, with which the direction or orientation reference is made. Like numbers refer to like elements throughout.

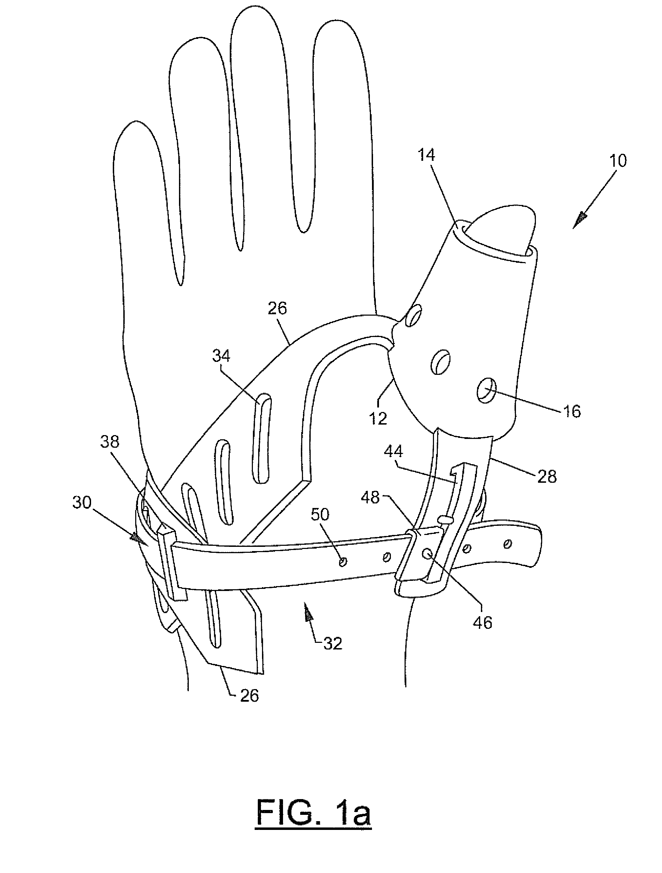

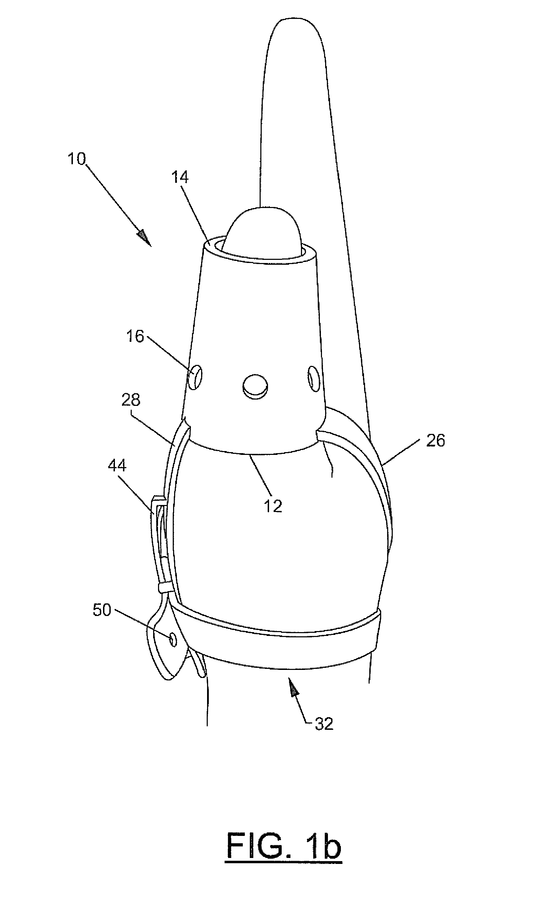

FIG. 1 (including FIGS. 1a and 1b) illustrates an apparatus according to example embodiments of the present invention fitted to a user's hand to reduce thumb sucking. It is to be understood that FIG. 1a may be considered as either a front or back drawing of the hand, that is, a view of the back of the hand or a view of the palm of the hand. It is further understood that representations shown in FIG. 1a may be identical for both the front and back of the hand and that the example embodiments shown in FIG. 1a may have essentially identical structure when viewed from the front or the back.

To further understand example embodiments of the present invention it should be noted that the term "digits" refers to the four fingers and the thumb, each being a "digit." The fifth digit (i.e., the little finger) is that digit which is furthest removed from the first digit (i.e., the thumb). The term "hand" is not limiting and can include the digits, the wrist section of the lower upper extremity, and the flat portion joining the digits and the wrist - the flat portion is often also referred to as the hand or palm.

The apparatus of example embodiments includes a vacuum-breaking device **10** generally shaped to be fitted or otherwise placed on a user's digit (finger or thumb) so that the device permits the flow of air from one end to the other so as to prevent the user from establishing a sucking vacuum when the respective digit is inserted into the user's mouth. The vacuum-breaking device may have any of a number of shapes to achieve its intended purpose. In various examples, the vacuum-breaking device may be in the shape of a hollow, closed or open-ended cylinder (cylindrical-shaped) or cone that generally conforms to the shape of a digit, and that may have or otherwise define ridges, vertical air passageways, tubes, apertures, air passages or the like to prevent establishment of the aforementioned sucking vacuum. A number of suitable examples are disclosed in the aforementioned '870 patent.

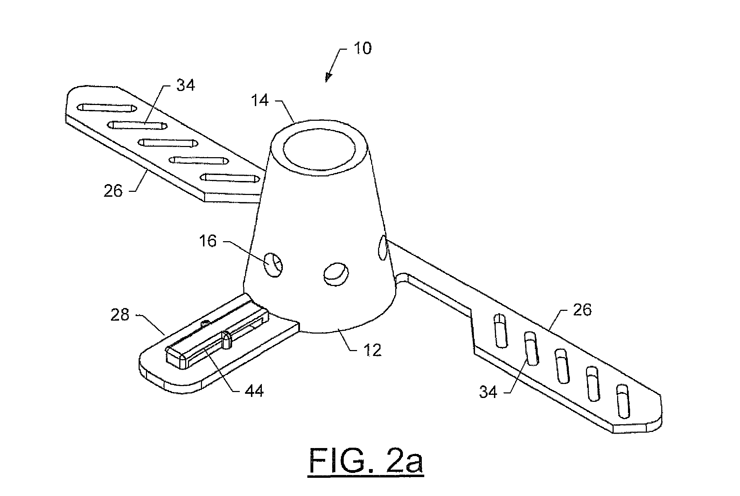

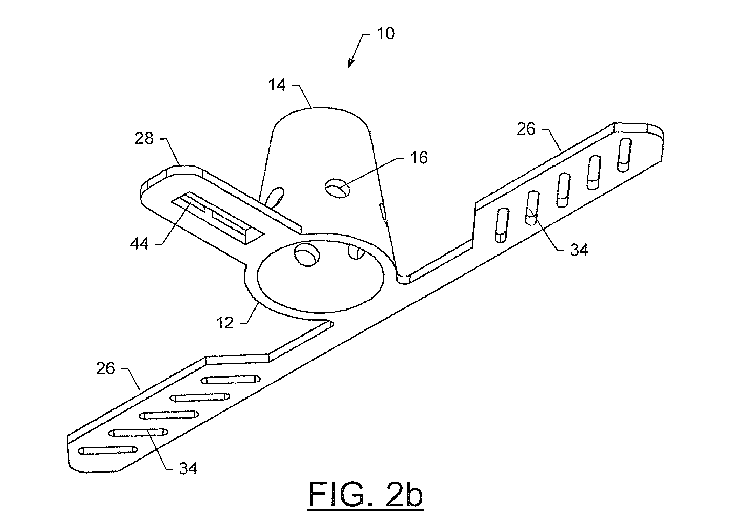

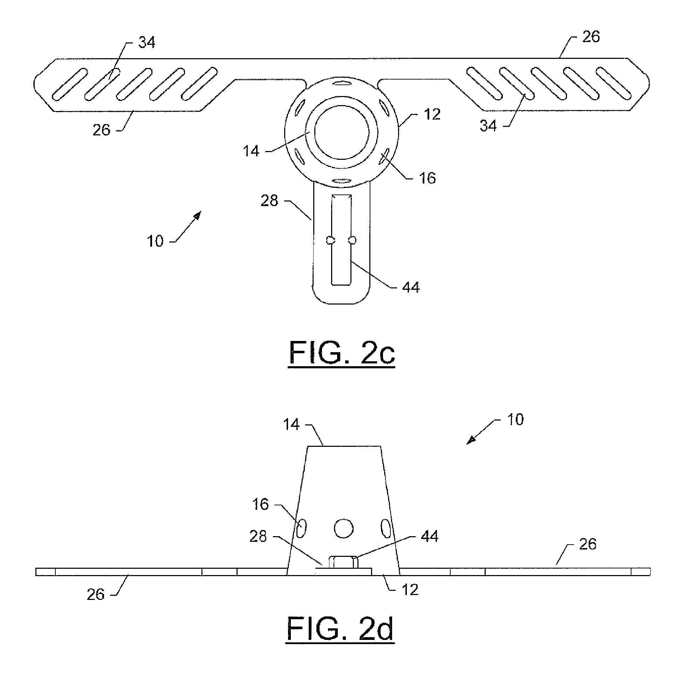

FIG. 2 (including FIGS. 2a-2d) illustrates various views of a vacuum-breaking device **10** of the apparatus of one example embodiment. As shown, the device of this example embodiment generally has a hollow, conical-frustum shape (sometimes referred to as a tapered cylinder or truncated cone). The device may include a base **12** and an opposing top **14** which in one example is smaller (e.g., diameter) than the base (or rather the base may be larger than the top). In one example, the vacuum-breaking device may include a bottom section extending from the base to an intermediate position between the base and top, and a top section extending from the intermediate position to the top. At least the base (or both the base and top) is of sufficient size to permit insertion of a user's digit (finger or thumb) through the base and into the device, thereby fitting the device on the user's digit. In this regard, the base may be positioned near the base of the user's digit while the top is positioned near the tip of the digit when (in an instance in which) the device is fitted on the digit.

As also shown in FIG. 2, the vacuum-breaking device **10** may define a number of apertures or air passages **16** around its circumference so as to prevent any vacuum that may otherwise be created when a digit fitted with the device is inserted into the mouth and the user sucks on the respective digit. To further facilitate preventing this sucking vacuum, in various examples, the base and top **12**, **14** may be further sized to permit the flow of air between the base and top through a passageway formed between the device and the user's digit inserted therein.

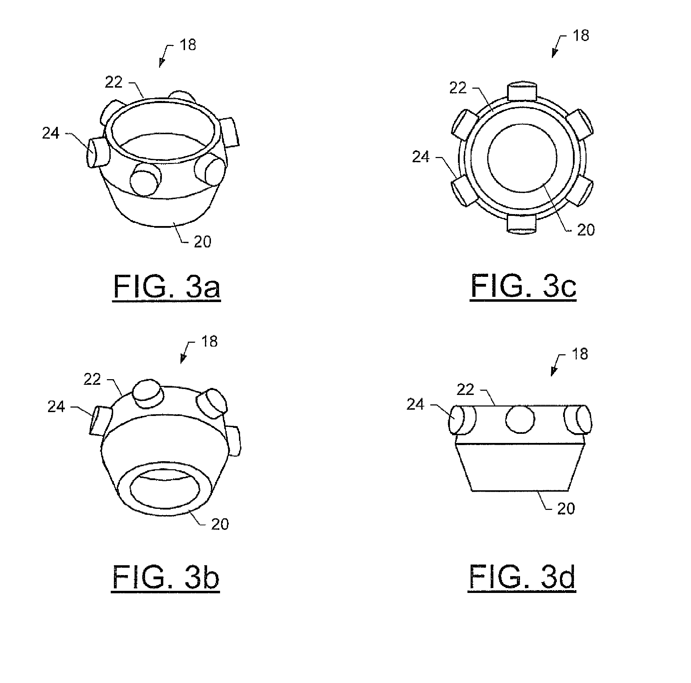

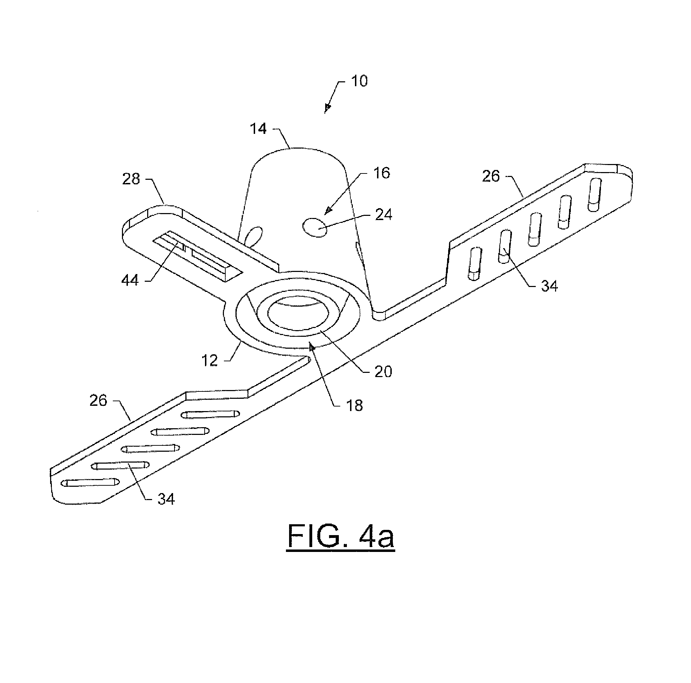

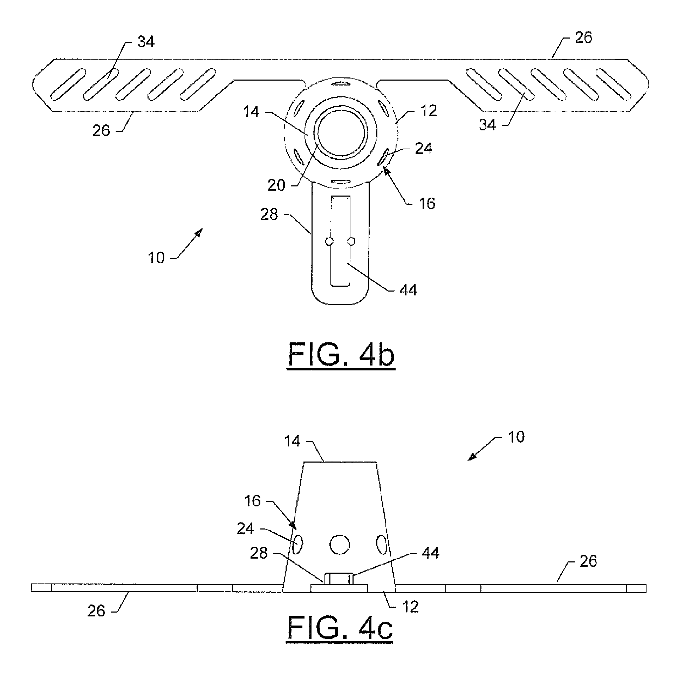

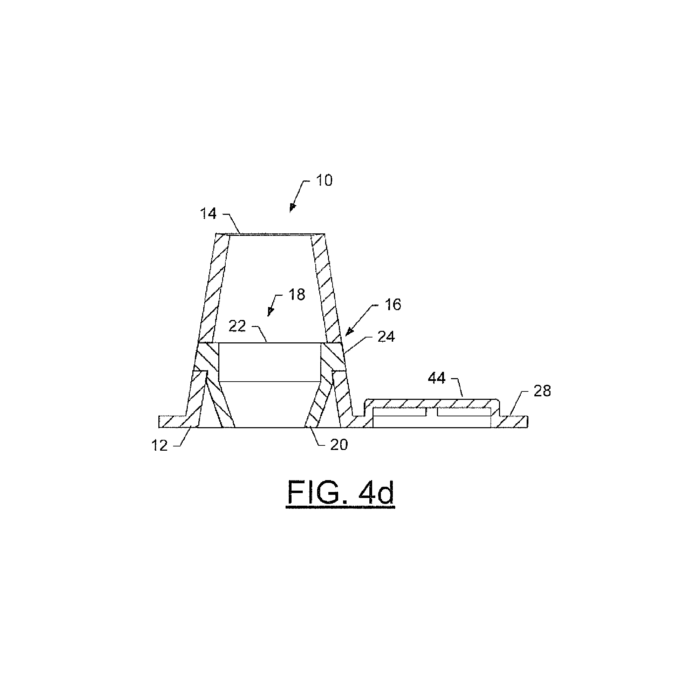

In various examples, the base **12** of the vacuum-breaking device **10** may be large enough that if not accounted for, a user may be able to bend their digit and slip it out of the device, thereby defeating the purpose of the apparatus. The apparatus of example embodiments may therefore optionally include a choke that may be fitted into or otherwise placed inside the bottom section of the vacuum-breaking device to reduce an effective size of the base of the device when the assembled device and choke is fitted onto the user's digit, thereby reducing the likelihood of the user being able to slip out of the device. FIG. 3 (including FIGS. 3a-3d) illustrates various views of a choke **18** of the apparatus of one example embodiment. FIG. 4 (including FIGS. 4a-4d) illustrates various views of an assembled vacuum-breaking device and choke of the apparatus of one example embodiment.

As shown and described herein, the vacuum-breaking device **10** and choke **18** may be constructed as separate components. In these example embodiments, the vacuum-breaking device and choke may be constructed of different material with different properties (e.g., hardness), and either may be replaceable without replacing the other. For example, the vacuum-breaking device may be made of a harder material than the choke which may enable the device to withstand biting or chewing by the user, and enable replacement of the device in instances in which the device becomes damaged due to prolonged use, biting, chewing or the like. It should be understood, however, that in other example embodiments, the vacuum-breaking device and choke may be constructed as a single component. In one example, the choke may be an extension of the vacuum-breaking device and fitted in such a way as to fold underneath it.

Similar to the vacuum-breaking device **10**, the choke **18** of one example generally has a hollow, conical-frustum shape but with a reverse taper as compared to the vacuum-breaking device. The choke may include a base **20** and an opposing top **22** which in one example is larger (e.g., diameter) than the base (or rather the base of the choke may be smaller than the top of the choke). Both the base and top of the choke are of sufficient size to permit insertion of the choke into the bottom section of the vacuum-breaking device, and insertion of a user's digit (finger or thumb) through the choke and into the device, thereby fitting the choke and device on the user's digit. The base of the choke may be generally smaller than that of the vacuum-breaking device to reduce an effective size of the base of the vacuum-breaking device, thereby reducing the likelihood of the user being able to slip out of the device, as indicated above. Similar to the vacuum-breaking device, in one example, the base of the choke may be positioned near the base of the user's digit while the top is positioned near an intermediate portion of the digit closer to the tip of the digit when the choke and device are fitted on the digit. Although not shown, in one example, the choke may define a number of apertures or air passages around its circumference so as to prevent any sucking vacuum that may otherwise be created when a digit fitted with the choke and device is inserted into the mouth and the user sucks on the respective digit.

The choke **18** may be fitted into the vacuum-breaking device **10** and secured in place in any of a number of different manners, and may fit completely within the device or extend out from the base **12** of the device. As shown in FIG. 4, in one example, the choke may include a plurality of protrusions **24** corresponding to a plurality of air passages **16** of the vacuum-breaking device **10.** The top **22** of the choke may have an outer dimension (e.g., diameter) approximately the same size or slightly smaller than a corresponding inner dimension of the vacuum-breaking device at its intermediate position, and as indicated above, the base **20** of the choke may be smaller than the base of the device. This may permit fitting the choke into the bottom section of the device with the protrusions extending out from respective air passages to thereby secure the choke in place.

Returning to FIG. 2 and with further reference to FIG. 4, a pair of longer semirigid straps **26** and a further optional shorter outer band **28** may extend from the base **12** of the vacuum-breaking device **10,** and may provide a means for securing the device (and choke) to the user's hand. The straps and band may be utilized to secure the device to the user's hand in any of a number of different manners. Suitable examples of manners by which the straps may be used to secure the device to the user's hand are shown and/or described in the aforementioned '870 patent. In other example embodiments, as shown in FIG. 1, the apparatus may include a retainer **30** configured to engage and cooperate with the pair of straps, and which may then engage and cooperate with a wristband **32** secured to the user's hand to thereby secure the straps and device to the user's hand.

In one example, the wristband **32** may be secured about the wrist by using a locking button such as that used in hospitals for securing identification bracelets on a person's wrist. Typically such locking buttons include a post and lock tab of such dimensions that when the post engages the lock tab, the post and lock tab lock together and are unable to be taken apart. When using the locking button type mechanism to fasten the wristband, it is anticipated that the wristband may be cut from the user's wrist and thrown away daily or even at shorter intervals. A more permanent device can be made in which the wristband is secured by buckle or other similar type securing mechanism.

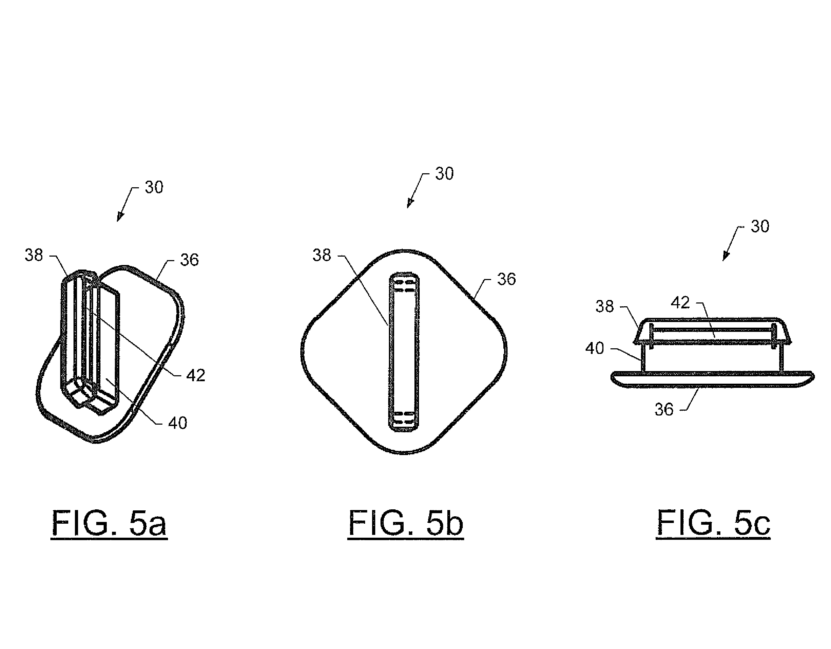

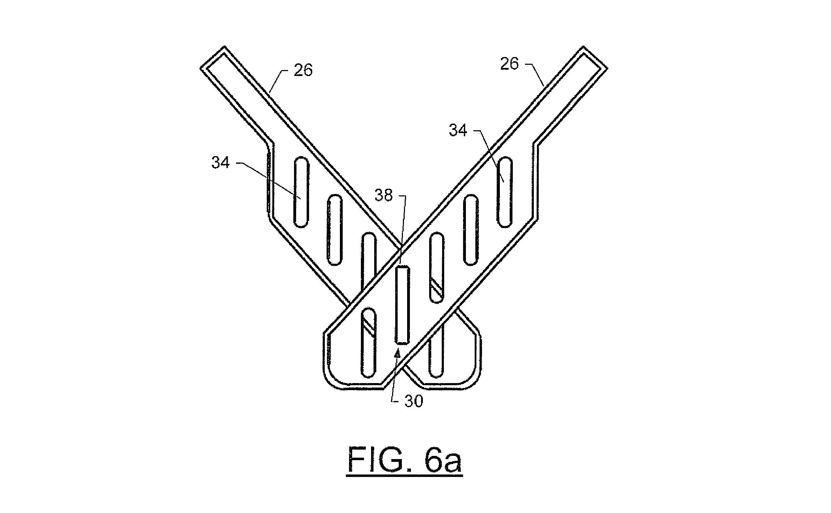

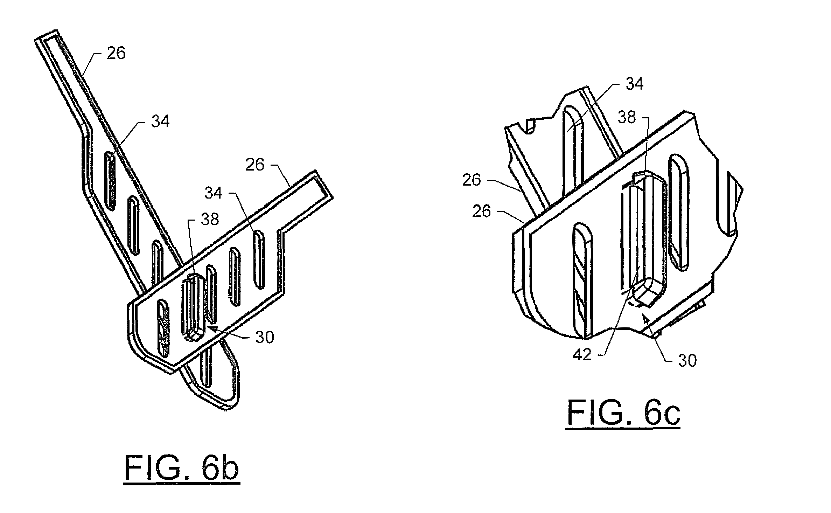

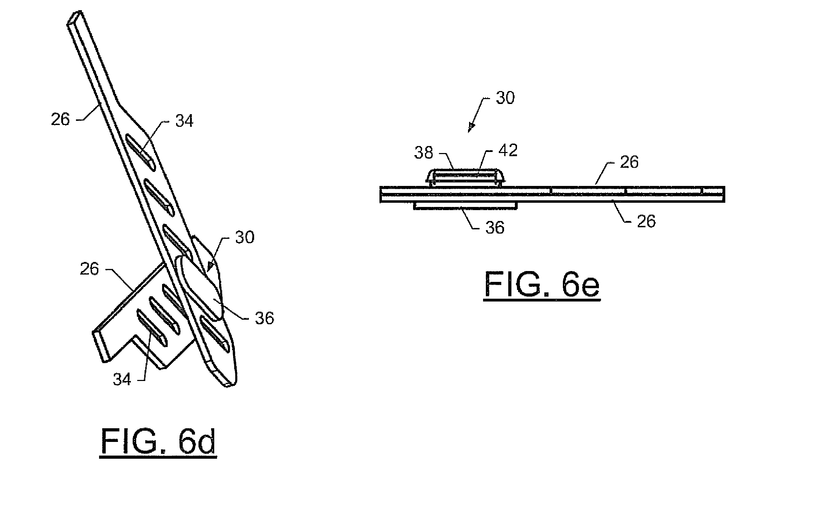

FIG. 5 (including FIGS. 5a-5c) and FIG. 6 (including FIGS. 6a-6e) illustrate various views of a retainer **30** and a portion of the pair of straps **26** of an example vacuum-breaking device **10** assembled with a retainer. The pair of straps may include one or more pairs of holes **34** (each strap including a hole of a pair of holes) for receiving the retainer. In one example, the holes of a pair of holes of the straps may be overlapped and secured with the retainer threaded through the overlapping holes and secured in place, as explained below. In instances in which the straps include more than one pair of holes, one of the pairs of holes may be selected according to the size of the user's hand and/or wrist, and the finger or thumb onto which the device and choke are fitted, to properly size the apparatus for being secured to the user's hand.

As shown in FIG. 5, according to one example, the retainer **30** may be a single-body retainer including a bottom section **36** and opposing top section **38** each of which is larger than the holes **34** of the straps **26,** and an intermediate section **40** between the bottom and top sections that is approximately the same size as or smaller than the respective holes. In the example shown, the bottom section is rectangular in shape and has a length and width larger than the largest dimension of the holes of the straps, and the top section is rectangular in shape and has a length longer than the largest dimension of the holes. In one example, holes of the wrist pad may have a profile approximately the same shape as the top section. In one example, the intermediate section of the retainer is approximately the same height as (or slightly longer than) the straps, and has a profile approximately the same size (or slightly smaller than) and shape as the holes of the straps. Either the top section of the retainer or holes of the straps may be slightly deformable to allow the retainer to be threaded through overlapping holes of the straps, and secured in place. In one example, the retainer may be rigid and the holes of the straps may be slightly deformable to allow the retainer to be threaded through overlapping holes of the straps, and secured in place.

Returning to FIG. 1, the apparatus may be secured to the user's wrist as or after its components, including the vacuum-breaking device **10** (and choke **18**) with straps **26** (and possibly also outer band **28**) is assembled and held together by the retainers **30**. The assembled vacuum-breaking device may be fitted to the user's digit with one of the straps extending along the back of the hand to the wrist area, and the other of the straps extending along the palm of the hand to the wrist area. The outer band may extend along the thumb side of the hand to the wrist area. As or after the vacuum-breaking device is fitted to the user's digit, appropriate holes **34** of the straps may be selected to provide the appropriate size and degree of tightness of the apparatus to the hand, e.g., at least sufficient to fit the user's wrist while preventing slippage of the digit out of the vacuum-breaking device. The respective holes of the straps may be overlapped and secured together by the retainer.

In one example, the retainer **30** may further define an aperture **42** through which the wristband **32** may be received. The outer band **28** may similarly include a loop **44** through which the wristband may be received. In one example, the outer band loop may be partitioned to allow sizing the apparatus on the user's hand, such as by allowing the wristband to be received through a partition of the loop closer to the user's thumb and above the wrist area. After passing through the retainer aperture and outer band loop, the wristband may be extended around the user's wrist and fastened to itself by means of a locking button that includes a post **46**, lock tab **48** and one or more holes **50.** The post and lock tab may be located near one end of the wristband, and the hole may be located at the other end of the wristband and placed on the post after the wristband surrounds the wrist. The appropriate hole of the wristband may be selected to provide the appropriate degree of tightness, e.g., at least sufficient to prevent the slippage of the wristband over the hand. The lock tab may then be folded over onto the lock post so as to lock the wristband in place.

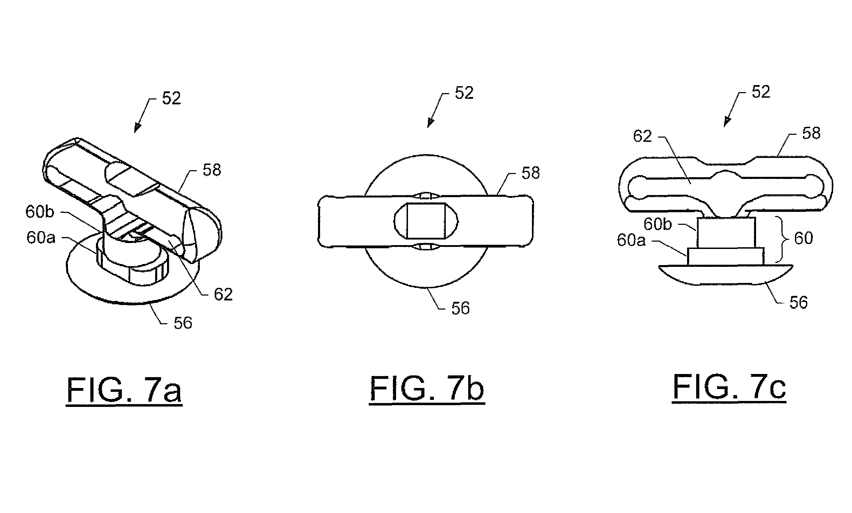

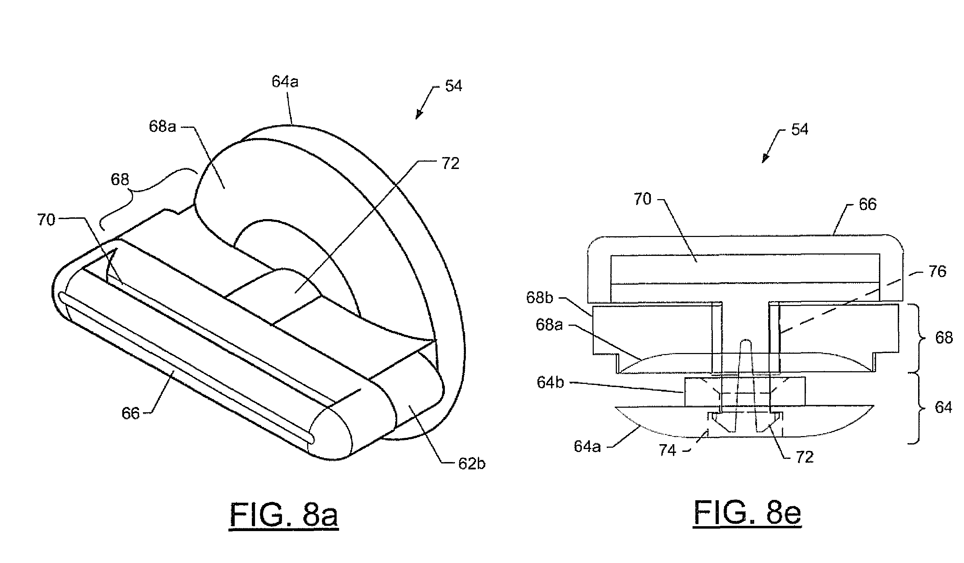

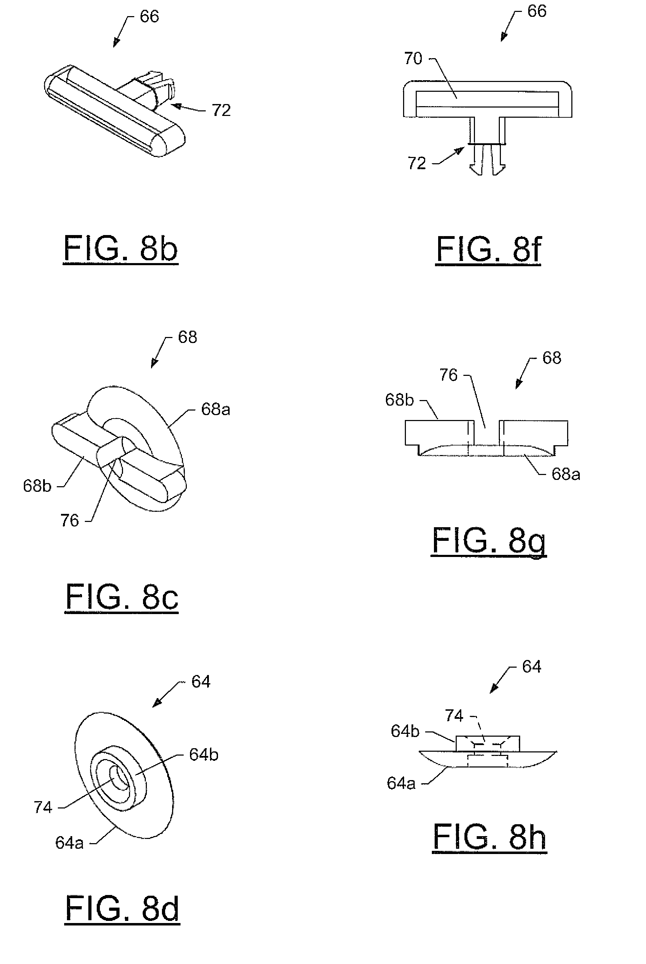

It may be possible that changes in the configurations to other than those shown or described could be used. FIGS. 7 and 8 illustrate retainers according to other example embodiments. FIG. 7 illustrates an example single-component retainer **52**, and FIG. 8 illustrates an example multi-component retainer **54**. The example retainers shown in FIGS. 7 and 8 may be particularly adapted for straps **26** with round holes, but it should be understood that the retainers may be equally adapted for holes of other shapes, including the elongated holes **34** shown and described herein.

As shown in FIG. 7 according to one example, the retainer **52** may include a bottom section **56** and opposing top section **58** each of which is larger than the holes of the straps **26**, and an intermediate section **60** between the bottom and top sections that is approximately the same size as or smaller than the respective holes. Similar to the retainer **30**, the retainer **52** may define an aperture **62** through which the wristband **32** may be received, such as in a similar manner as described above. In the example shown, the bottom section is circular in shape and has a diameter larger than the largest dimension of the holes of the straps, and the top section is rectangular in shape and has a length longer than the largest dimension of the holes.

In one example, the intermediate section **60** of the retainer **52** includes two subsections, a bottom intermediate subsection **60a** and a top intermediate subsection **60b.** In one example, either or both of the bottom intermediate subsection or top intermediate section is approximately the same height as (or slightly longer than) the straps **26** and has a profile approximately the same size (or slightly smaller than) and shape as the holes of the straps.

Either or both of the bottom or top sections **56**, **58** of the retainer **52** may be removable or deformable to allow the retainer to be threaded through overlapping holes of the straps **26**, and secured in place. As shown in FIG. 7, for example, the top section of the retainer may generally have a horizontal orientation extending generally perpendicular to the height of the intermediate section **60.** The retainer may be constructed so that when the sides of the top section are squeezed inward, the top section deforms to change its orientation to a generally vertical orientation extending generally parallel to the height of the intermediate section, thereby allowing the top section to be threaded through the overlapping holes of the straps. Then, when the sides of the top section of the retainer are released, the retainer may return to a generally vertical orientation, thereby securing the retainer to the straps.

As shown in FIG. 8, according to one example, the multi-component retainer **54** may include a bottom section **64**, top section **66** and intermediate section **68.** The sections may be separate components that, when assembled and secured together, form the retainer in which the top section opposes the bottom section and the intermediate section is interposed therebetween. Again similar to the retainer **30**, the retainer **54** may define an aperture **70** through which the wristband **32** may be received, such as in a similar manner as described above. In one example, the bottom section includes two subsections, a base bottom subsection **64a** and a top bottom subsection **64b**. Similarly, in one example, the intermediate section of the retainer includes two subsections, a bottom intermediate subsection **68a** and a top intermediate subsection **68b**.

Any one or more of the bottom section **64**, top section **66** or intermediate section **68** may be approximately the same size as or larger than the holes of the straps **26**. In the example shown, the top section has a profile approximately the same size (or slightly larger than) and shape as the holes of the straps **26**. In this example, the bottom intermediate subsection **68a** is circular in shape and has a diameter larger than the largest dimension of the holes of the straps; and the top intermediate subsection **68b** is approximately the same height as (or slightly longer than) the straps and has a profile approximately the same size (or slightly larger than) and shape as the holes of the straps.

The top section **66** of the retainer **54** may include an axial post **72**, and the each of the bottom and intermediate sections **64**, **68** may define a respective axial hole **74**, **76** for receiving the post. As shown, the hole of the bottom section and the post may be shaped so that when the respective hole receives the post, the post locks in place, thereby forming a tapered or cantilevered snap fit latch. To assemble the straps **26** of the vacuum-breaking device **10**, similar to before, the holes of the straps **26** may be overlapped. The intermediate section of the retainer may be situated underneath one of the straps such that its top intermediate section **68b** fits within the hole of the strap from underneath. The post of the top section may be threaded through the holes of the bottom and intermediate sections, and thereby through the holes of the straps, and lock into place at the bottom section. The retainer may thereby be secured to the straps.

Many modifications and other embodiments of the invention will come to mind to one skilled in the art to which this invention pertains having the benefit of the teachings presented in the foregoing descriptions and the associated drawings. For example, it is to be understood that various means for fastening the various components of this invention together may be used or that various parts of the invention can be assembled as a single integral unit. It should therefore be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. An apparatus for discouraging a user from sucking on a digit of the user's hand, the apparatus comprising:
a hollow vacuum-breaking device (10) including a base (12) and defining one or more air passages (16), the vacuum-breaking device (10) being sized to permit insertion of the user's digit into the vacuum-breaking device (10) so that the base of the vacuum-breaking device (10) is nearer to the base of the digit than the tip thereof, the air passages (16) of the vacuum-breaking device (10) preventing the user from establishing a sucking vacuum when the respective digit including the vacuum-breaking device (10) is inserted into the user's mouth;
a pair of straps (26) extending from the vacuum-breaking device (10), **characterised in that** the straps of the pair of straps (26) defining respective pluralities of holes (34) along their length, a pair of the holes (34) including a hole (34) of each of the pluralities being selectable according to a size of the user's hand, the straps (26) being configured to cross when the holes (34) of the pair of holes overlap; and
a retainer (30) configured to extend through overlapping holes (34) of the straps (26) to thereby secure the straps (26) together, the retainer (30) defining an aperture (42) for receiving a wristband (32) securable to the user's wrist to secure the straps (26) to the user's wrist, the vacuum-breaking device (10) thereby being securable to the user's hand.

2. The apparatus of Claim 1, wherein the retainer (30) is rigid, and the holes (34) of the straps (26) are slightly deformable to allow the retainer (30) to be threaded through the overlapping holes (34) and secured in place.

3. The apparatus of Claim I or 2, wherein the retainer comprises:
a bottom section (56) and an opposing top section (58) each of which is larger than the holes (34) of the straps (26); and
an intermediate section (60) between the bottom and top sections that is approximately the same size as or smaller than the holes (34) of the straps (26).

4. The apparatus of Claim 3, wherein the top section (58) is inwardly squeezable from a first orientation to a second orientation to allow the top section (58) to be threaded through the overlapping holes (34), the top section (58) being capable of thereafter returning to the first orientation to secure the retainer in place.

5. The apparatus of any preceding claim, further comprising:
a hollow choke (18) including a base that is fixed in size and smaller than that of the vacuum-breaking device (10),
wherein the vacuum-breaking device (10) and choke (18) are sized to permit insertion of the choke (18) into a bottom section of the vacuum-breaking device (10) proximate the base thereof, the vacuum-breaking device (10) and choke (18) thereby being assembled, and
wherein the vacuum-breaking device (10)and choke (18) are also sized to permit insertion of the user's digit into the assembled vacuum-breaking device (10) and choke (18) so that the bases of the vacuum-breaking device (10) and choke (18) are nearer to the base of the digit than the tip thereof.

6. The apparatus of Claim 5, wherein the vacuum-breaking device (10) has a conical-frustum shape including the base (12) and an opposing top (14) which has a diameter smaller than that of the base (12) of the vacuum-breaking device (10), and
wherein the choke (18) has a conical-frustum shape including the base (20) and an opposing top (22) which has a diameter larger than that of the base (20) of the choke (18), the diameter of the base (20) of the choke (18) being smaller than that of the base (12) of the vacuum-breaking device (10).

7. The apparatus of Claim 5 or 6, wherein the choke (18) includes a plurality of protrusions (24) corresponding to a plurality of air passages (16) of the vacuum-breaking device (10), the protrusions (24) being configured to extend out from respective air passages of the assembled vacuum-breaking device (10) and choke (18) to thereby secure the choke (18) in place.

## Patentansprüche

1. Vorrichtung für das Abhalten eines Benutzers vom Lutschen am Finger der Hand des Benutzers, wobei die Vorrichtung aufweist:
eine hohle Vakuumunterbrechungsvornchtung (10), einschließlich einer Basis (12) und einen oder mehrere Luftdurchgänge (16) definierend, wobei die Vakuumunterbrechungsvorrichtung (10) bemessen ist, um das Einführen des Fingers des Benutzers in die Vakuumunterbrechungsvorrichtung (10) zu gestatten, so dass die Basis der Vakuumunterbrechungsvornchtung (10) näher an der Fingerbasis ist als an dessen Spitze, wobei die Luftdurchgänge (16) der Vakuumunterbrechungsvorrichtung (10) verhindern, dass der Benutzer das Ansaugen eines Vakuums aufnimmt, wenn der jeweilige Finger, einschließlich der Vakuumunterbrechungsvorrichtung (10), in den Mund des Benutzers eingeführt wird;
ein Paar Riemen (26), die sich von der Vakuumunterbrechungsvorrichtung (10) erstrecken, **dadurch gekennzeichnet, dass** die Riemen des Paares der Riemen (26) eine jeweilige Vielzahl von Löchern (34) entlang ihrer Länge definieren, wobei ein Paar der Löcher (34) ein Loch (34) einer jeden der Vielzahl umfasst, das entsprechend einer Größe der Hand des Benutzers wählbar ist, wobei die Riemen (26) so ausgebildet sind, dass sie sich kreuzen, wenn sich die Löcher (34) des Paares der Löcher überlappen; und
einen Halter (30), der ausgebildet ist, um sich durch die sich überlappenden Löcher (34) der Riemen (26) zu erstrecken, um dadurch die Riemen (26) miteinander zu sichern, wobei der Halter (30) eine Öffnung (42) für das Aufnehmen eines Armbandes (32) definiert, das am Handgelenk des Benutzers gesichert werden kann, um die Riemen (26) am Handgelenk des Benutzers zu sichern, wodurch die Vakuumunterbrechungsvorrichtung (10) an der Hand des Benutzers gesichert werden kann.

2. Vorrichtung nach Anspruch 1, bei der der Halter (30) starr ist, und wobei die Löcher (34) der Riemen (26) leicht verformbar sind, um zu gestatten, dass der Halter (30) durch die sich überlappenden Löcher (34) eingezogen und an Ort und Stelle gesichert wird.

3. Vorrichtung nach Anspruch 1 oder 2, bei der der Halter aufweist:
einen unteren Abschnitt (56) und einen entgegengesetzten oberen Abschnitt (58), wobei ein jeder davon größer ist als die Löcher (34) der Riemen (26); und
einen Zwischenabschnitt zwischen dem unteren und dem oberen Abschnitt, der annähernd die gleiche Größe aufweist wie die oder kleiner ist als die Löcher (34) der Riemen (26).

4. Vorrichtung nach Anspruch 3, bei der der obere Abschnitt (58) nach innen aus einer ersten Ausrichtung in eine zweite Ausrichtung gepresst werden kann, damit der obere Abschnitt (58) durch die sich überlappenden Löcher (34) eingezogen werden kann, wobei der obere Abschnitt (58) danach in die erste Ausrichtung zurückgeführt werden kann, um den Halter an Ort und Stelle zu sichern.

5. Vorrichtung nach einem der vorhergehenden Ansprüche, die außerdem aufweist:
eine hohle Drossel (18), einschließlich einer Basis, die hinsichtlich der Größe unveränderlich und kleiner als die der Vakuumunterbrechungsvorrichtung (10) ist;
wobei die Vakuumunterbrechungsvorrichtung (10) und die Drossel (18) bemessen sind, um das Einsetzen der Drossel (18) in einen unteren Abschnitt der Vakuumunterbrechungsvorrichtung (10) in unmittelbarer Nähe von deren Basis zu gestatten, wodurch die Vakuumunterbrechungsvorrichtung (10) und die Drossel (18) dadurch zusammengebaut werden; und
wobei die Vakuumunterbrechungsvorrichtung (10) und die Drossel (18) ebenfalls bemessen sind, um das Einsetzen des Fingers des Benutzers in die zusammengebaute Vakuumunterbrechungsvorrichtung (10) und Drossel (18) zu gestatten, so dass die Basis der Vakuumunterbrechungsvorrichtung (10) und die Basis der Drossel (18) näher an der Fingerbasis als an dessen Spitze sind.

6. Vorrichtung nach Anspruch 5, bei der die Vakuumunterbrechungsvorrichtung (10) eine Kegelstumpfform aufweist, einschließlich der Basis (12) und einer entgegengesetzten Oberseite (14), die einen Durchmesser aufweist, der kleiner ist als der der Basis (12) der Vakuumunterbrechungsvorrichtung (10); und
wobei die Drossel (18) eine Kegelstumpfform aufweist, einschließlich der Basis (20) und einer entgegengesetzten Oberseite (22), die einen Durchmesser aufweist, der größer ist als der der Basis (20) der Drossel (18), wobei der Durchmesser der Basis (20) der Drossel (18) kleiner ist als der der Basis (12) der Vakuumunterbrechungsvorrichtung (10).

7. Vorrichtung nach Anspruch 5 oder 6, bei der die Drossel (18) eine Vielzahl von Vorsprüngen (24) entsprechend einer Vielzahl von Luftdurchgängen (16) der Vakuumunterbrechungsvorrichtung (10) umfasst, wobei die Vorsprünge (24) so ausgebildet sind, dass sie sich von den jeweiligen Luftdurchgängen der zusammengebauten Vakuumunterbrechungsvorrichtung (10) und Drossel (18) aus erstrecken, um dadurch die Drossel (18) an Ort und Stelle zu sichern.

## Revendications

1. Appareil destiné à décourager un utilisateur de sucer un doigt de la main de l'utilisateur, l'appareil comprenant :
un dispositif casse-vide creux (10), englobant une base (12) et définissant un ou plusieurs passages d'air (16), le dispositif casse-vide (10) étant dimensionné de sorte à permettre l'insertion du doigt de l'utilisateur dans un dispositif casse-vide (10), la base du dispositif casse-vide (10) étant ainsi plus proche de la base du doigt que de la pointe de celui-ci, les passages d'air (16) du dispositif casse-vide (10) empêchant l'utilisateur d'établir un vide d'aspiration lorsque le doigt respectif englobant le dispositif casse-vide (10) est inséré dans la bouche de l'utilisateur ;
une paire de sangles (26), s'étendant à partir du dispositif casse-vide (10), **caractérisé en ce que** les sangles de la paire de sangles (26) définissent plusieurs trous (34) le long de leur longueur, une paire des trous (34) englobant un trou (34) de chacun des plusieurs trous, pouvant être sélectionné en fonction d'une taille de la main de l'utilisateur, les sangles (26) étant configurées de sorte à se croiser lors d'un chevauchement des trous (34) de la paire de trous ; et
un élément de retenue (30), configuré de sorte à s'étendre à travers les trous à chevauchement (34) des sangles (26) pour fixer ainsi les sangles (26) l'une à l'autre, l'élément de retenue (30) définissant une ouverture (42) destinée à recevoir un serre-poignet (32) pouvant être fixé sur le poignet de l'utilisateur pour fixer les sangles (26) sur le poignet de l'utilisateur, le dispositif casse-vide (10) pouvant ainsi être fixé sur la main de l'utilisateur.

2. Appareil selon la revendication 1, dans lequel l'élément de retenue (30) est rigide, les trous (34) des sangles (26) pouvant être légèrement déformés pour permettre le filetage de l'élément de retenue (30) à travers les trous à chevauchement (34) et la fixation dans sa position.

3. Appareil selon les revendications 1 ou 2, dans lequel l'élément de retenue comprend :
une section inférieure (56) et une section supérieure opposée (58), chacune de ces sections étant plus grande que les trous (34) des sangles (26) ; et
une section intermédiaire (60) entre les sections inférieure et supérieure, ayant à peu près la même taille que les trous (34) des sangles (26) ou une taille inférieure à ceux-ci.

4. Appareil selon la revendication 3, dans lequel la section supérieure (58) peut être comprimée vers l'intérieur, d'une première orientation vers une deuxième orientation, pour permettre le filetage de la section supérieure (58) à travers les trous à chevauchement (34), la section supérieure (58) étant capable de retourner ensuite vers la première orientation pour fixer l'élément de retenue dans sa position.

5. Appareil selon l'une quelconque des revendications précédentes, comprenant en outre :
un moyen d'étranglement creux (18), englobant une base ayant une taille fixe, inférieure à celle du dispositif casse-vide (10) ;
dans lequel le dispositif casse-vide (10) et le moyen d'étranglement (18) sont dimensionnés de sorte à permettre l'insertion du moyen d'étranglement (18) dans une section inférieure du dispositif casse-vide (10), à proximité de la base de celui-ci, le dispositif casse-vide (10) et le moyen d'étranglement (18) étant ainsi assemblés ; et
dans lequel le dispositif casse-vide (10) et le moyen d'étranglement (18) sont en outre dimensionnés de sorte à permettre l'insertion du doigt de l'utilisateur dans le dispositif casse-vide (10) et le moyen d'étranglement (18) assemblés, les bases du dispositif casse-vide (10) et du moyen d'étranglement (18) étant ainsi plus proches de la base du doigt que de la pointe de celui-ci.

6. Appareil selon la revendication 5, dans lequel le dispositif casse-vide (10) a une forme en tronc de cône englobant la base (12) et une partie supérieure opposée (14), ayant un diamètre inférieur à celui de la base (12) du dispositif casse-vide (10) ; et
dans lequel le moyen d'étranglement (18) a une forme en tronc de cône englobant la base (20) et une partie supérieure opposées (22), ayant un diamètre supérieur à celui de la base (20) du moyen d'étranglement (18), le diamètre de la base (20) du moyen d'étranglement (18) étant inférieur à celui de la base (12) du dispositif casse-vide (10).

7. Appareil selon les revendications 5 ou 6, dans lequel le moyen d'étranglement (18) englobe plusieurs saillies (24) correspondant à plusieurs passages d'air (16) du dispositif casse-vide (10), les saillies (24) étant configurées de sorte à s'étendre vers l'extérieur à partir des passages d'air respectifs du dispositif casse-vide (10) et du moyen d'étranglement (18) assemblés, pour fixer ainsi le moyen d'étranglement (18) dans sa position.
